## Europäisches Patentamt
## European Patent Office
## Office européen des brevets

(11) Numéro de publication: **0 144 275**
**B1**

(12) # FASCICULE DE BREVET EUROPÉEN

(45) Date de publication du fascicule du brevet:
**08.03.89**

(51) Int. Cl.⁴: **G 01 N 21/90**

(21) Numéro de dépôt: **84402514.8**

(22) Date de dépôt: **06.12.84**

(54) **Dispositif d'éclairement d'un liquide contenu dans un récipient, en vue de son contrôle.**

(30) Priorité: **07.12.83 FR 8319561**

(43) Date de publication de la demande:
**12.06.85 Bulletin 85/24**

(45) Mention de la délivrance du brevet:
**08.03.89 Bulletin 89/10**

(84) Etats contractants désignés:
**AT BE CH DE FR GB IT LI LU NL SE**

(56) Documents cité:
**EP-A-0 066 143**
**DE-C-682 869**
**US-A-2 436 262**

(73) Titulaire: **AEROSPATIALE SOCIETE NATIONALE INDUSTRIELLE, 37, Boulevard de Montmorency, F-75781 Paris Cédex 16 (FR)**
Titulaire: **SANOFI, 40, Avenue George V, F-75008 Paris (FR)**

(72) Inventeur: **Godard, Jean-Henri, 14, rue Duluc Gironde, F-33000 Bordeaux (FR)**
Inventeur: **Ometz, Pierre 7 Villepreux Village, Saint-Aubin de Medoc Gironde, F-33160 Saint Medard en Jalles (FR)**
Inventeur: **Labrador, Jacques, 24, rue d'Hendaye Haute-Garonne, F-31240 L'Union (FR)**

(74) Mandataire: **Lepeudry-Gautherat, Thérèse, ARMENGAUD JEUNE CABINET LEPEUDRY 6, rue du Fg. St-Honoré, F-75008 Paris (FR)**

## Description

L'invention concerne un dispositif d'éclairage d'un liquide transparent contenu dans un récipient transparent au rayonnement utilisé, et dont les parois latérales sont parallèles à un axe longitudinal, en vue de son contrôle. Elle concerne notamment le contrôle des liquides alimentaires, des solutés injectables et des fabrications pharmaceutiques se présentant notamment sous forme d'ampoules ou flacons de produits injectables.

Le contrôle consiste en la détection de la présence éventuelle de particules solides en suspension naturelle ou provoquée dans le produit liquide.

Il s'effectue généralement en éclairant le liquide à partir d'une source de lumière placée latéralement ou au fond du récipient. L'observation peut se faire dans l'axe de la source, l'occultation d'une fraction de la lumière par les particules provoquant leur détection. Elle peut se faire en observant suivant un axe différent de celui de la source, les réflexions, réfractions, diffractions provoquées par les éventuelles particules.

Dans le cas d'un éclairage par le fond, des dispositifs connus utilisent généralement une source de lumière et des moyens permettant de faire pénétrer la lumière par le fond du récipient. Toutefois, ces dispositifs connus présentent plusieurs inconvénients liés au fait qu'ils ne tiennent pas compte de la déformation du faisceau lumineux par le récipient lui-même. En effet, les parois du récipient se comportent comme des dioptres air-liquide qui modifient le faisceau lumineux; elles le font par exemple converger ou diverger en fonction notamment de la courbure des parois. Il s'ensuit un faisceau lumineux déformé par la paroi du récipient, qui n'éclaire pas de façon homogène le liquide à contrôler, c'est-à-dire que l'éclairement produit au sein du liquide est variable d'un point à un autre. Par ailleurs, ces dispositifs connus éclairent sans distinction le récipient et le liquide. Il en résulte que les défauts ou les inscriptions existant sur les parois du récipient, et ainsi éclairés, sont la source d'erreurs de détection, ces défauts interférant avec le faisceau lumineux de la même façon que les particules en suspension dans le liquide et pouvant être ainsi confondus avec elles. L'élimination de ces détections erronées peut être obtenue en diminuant le niveau d'éclairement produit par la source, ce qui a pour conséquence de diminuer de manière importantes les signaux lumineux émis par les particules et servant à leur détection.

Des dispositifs d'éclairage connus de l'art antérieur sont décrits dans les documents EP-A-0 086 143 et US-A-2 436 262.

Selon l'invention, tous les inconvénients précités sont évités grâce à un dispositif d'éclairement tel que décrit dans la revendication 1.

Ainsi, selon l'un des buts de la présente invention, il est tenu compte des caractéristiques de dimensions et de forme du récipient, ainsi que de la nature du liquide et de sa disposition à l'intérieur du récipient, pour produire, à partir du dispositif d'éclairement, un faisceau lumineux tel, qu'en traversant le fond du récipient, il soit modifié de façon à assurer un éclairage optimal du liquide.

De préférence, la source lumineuse est une source secondaire, dont les dimensions sont compatibles avec celles du récipient et qui est formée à partir d'une source primaire, au travers d'un premier système optique, les moyens de mise en forme du faisceau issu de la source secondaire étant constitués par un second système optique formant, en coopération avec le fond lui-même, l'image de la source secondaire située au voisinage de la surface terminale du liquide, ou au-delà.

L'utilisation d'une source lumineuse primaire et d'une source secondaire, précédemment définie, permet de dissocier avantageusement les problèmes liés à la quantité de lumière à produire et les problèmes liés à la formation d'une image de la source secondaire, à travers le second système optique et le récipient: la source primaire, ou source chaude, est étudiée et dimensionnée en fonction de l'énergie lumineuse nécessaire à un éclairement convenable de la masse de liquide contenu dans le récipient, tandis que la source secondaire, ou source froide, est étudiée de façon plus précise en fonction des dimensions et de la forme de la section du récipient. L'étude du dispositif montre que pour obtenir une adaptation satisfaisante, la source secondaire doit avoir des dimensions comparables avec celles du récipient, c'est-à-dire qui sont inférieures à celles-ci, dans un rapport de un à cinq. De plus, pour que l'adaptation de la forme du faisceau à celle du récipient soit optimale, la surface apparente de la source secondaire, dans l'axe du dispositif d'éclairement, doit être homothétique de la section droite du récipient. Ceci peut être réalisé, soit en choisissant la forme même de la source secondaire, soit en utilisant un diaphragme ayant la forme requise et qui peut être placé au niveau de la source, ou en tout point où se forme une image de celle-ci.

Le premier système optique peut être constitué par tout moyen permettant la concentration de la lumière issue de la source primaire sur la source secondaire: il peut être constitué par un miroir unique, ou d'une ou plusieurs lentilles, ou encore, de l'association de ces deux moyens.

Le second groupe optique peut, lui aussi, être constitué par une ou plusieurs lentilles, ou par un ou plusieurs miroirs de courbures appropriées.

Un premier mode de réalisation de l'invention concerne des ampoules de faible contenance, telles que des ampoules pharmaceutiques de quelques millilitres. Le premier système optique comprend, avantageusement dans ce cas, une fibre optique de section homothétique de celle du récipient, dont l'entrée reçoit le faisceau issu de la source primaire et dont la sortie constitue la source secondaire, et il est prévu un diaphragme de section sensiblement égale à la section intérieure du récipient, qui est disposé entre le second système optique et le fond du récipient.

Le diaphragme peut être constitué par un moyen de positionnement du récipient. De préférence, les ampoules sont disposées verticalement, fond en bas, la surface terminale précitée étant alors constituée par la surface libre du liquide.

Un second mode de réalisation de l'invention concerne des flacons de grandes contenance, dont le diamètre est compris entre quelques centimètres et une dizaine de centimètres, tels que des flacons de solutés massifs ou de liquides alimentaires. Le principe de l'invention s'applique alors différemment car il faut adapter la forme et les dimensions des sources primaire et secondaire à la forme et aux dimensions de ce type de récipient. Il faut, d'autre part, maintenir le niveau d'éclairement, donc augmenter le flux émis par la source primaire et, par conséquent, augmenter la surface de celle-ci.

On peut, dans certains cas, maintenir le dispositif précité relatif aux petites ampoules, et augmenter le diamètre de la fibre. Dans les cas où une telle possibilité n'est ni envisageable ni souhaitable, il faut utiliser un autre dispositif.

Dans un tel dispositif, le premier système optique concentre le faisceau sur un premier diaphragme de section homothétique de celle du récipient, qui est disposé entre les deux systèmes optiques et qui constitue la source secondaire, et il est prévu un second diaphragme de section sensiblement égale à la section intérieure du récipient, qui est disposé entre le second système optique et le fond du récipient.

Le premier diaphragme peut éventuellement être confondu, tant en position qu'en diamètre, avec le support du dernier élément optique du premier système optique. Quant au second diaphragme, il peut être constitué par un moyen de positionnement du récipient.

De préférence, le récipient ou flacon est disposé verticalement, fond en haut, la surface terminale précitée étant alors constituée par la surface d'appui du liquide sur une autre paroi d'extrémité du récipient.

Les moyens de mise en forme du faisceau peuvent comprendre un miroir plan de renvoi, qui est disposé en travers du faisceau, entre le premier groupe optique et le fond du récipient. Un tel miroir permet de renvoyer le faisceau lumineux provenant de la source primaire selon, par exemple, une direction décalée de 90° par rapport à sa direction d'incidence, de façon à limiter notamment l'encombrement vertical du montage de contrôle des récipients.

La surface terminale du liquide précitée est celle par laquelle le faisceau lumineux sort du liquide, après avoir traversé celui-ci. Dans le cas des petites ampoules disposées fond en bas, le faisceau pénètre dans le liquide par sa surface de contact avec le fond de l'ampoule et en ressort par sa surface libre, laquelle constitue donc la surface terminale du liquide. Dans le cas des grands flacons disposés fond en haut, le faisceau lumineux pénètre dans le liquide par sa surface libre faisant face au fond du flacon, et il en ressort par sa surface de contact avec le goulot du flacon, laquelle constitue alors la surface terminale du liquide.

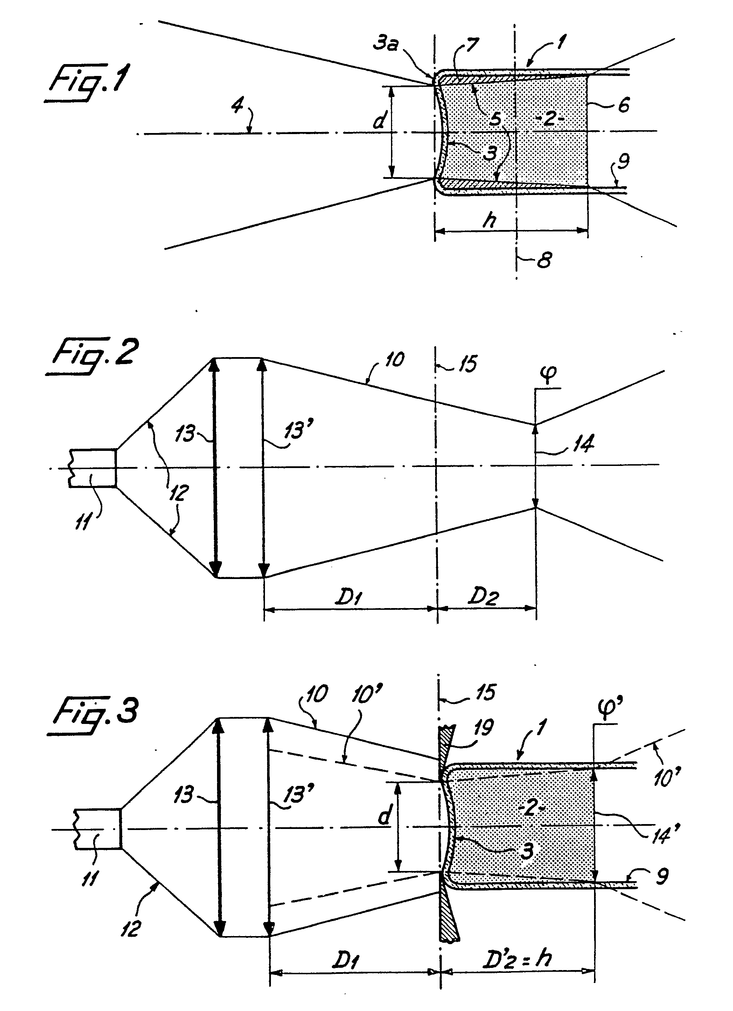

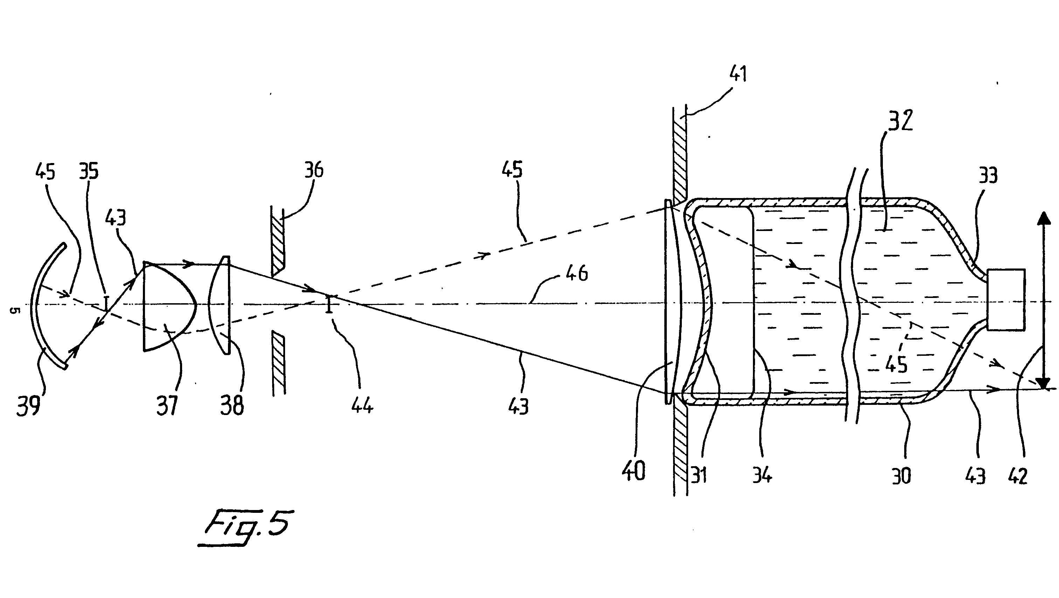

La description qui suit s'attachera à illustrer les deux modes de réalisation de l'invention évoqués précédemment, à titre d'exemples non limitatifs. Les figures 1 à 4 annexées, concernent un dispositif d'éclairement de petites ampoules, alors que la figure 5 concerne un dispositif d'éclairement de grands flacons. Plus précisément:

La figure 1 représente le trajet théorique du faisceau lumineux adapté à une ampoule cylindrique à fond concave.

La figure 2 représente le faisceau lumineux produit par le dispositif d'éclairement de l'invention et permettant l'éclairement d'une ampoule comme défini en figure 1.

La figure 3 représente le faisceau lumineux obtenu, lorsque l'ampoule est disposée devant le dispositif d'éclairement.

La figure 4 illustre une réalisation concrète du dispositif d'éclairement des figures précédentes.

La figure 5 illustre une réalisation concrète d'un dispositif d'éclairement pour grands flacons.

Dans ce qui suit, il sera montré comment a été appliqué le principe de l'invention à l'éclairement d'ampoules de forme générale cylindrique et possédant un fond concave, qui contiennent un liquide pharmaceutique à contrôler, l'éclairement s'effectuant à partir du fond. La figure 1 représente partiellement une telle ampoule 1 contenant un liquide 2. Cette ampoule pourrait ne pas être cylindrique. L'important est que ses parois latérales soient parallèles à un axe longitudinal 4. Pour faciliter la représentation, cette ampoule 1, reposant en principe verticalement sur un support, fond en bas, est représentée ici horizontalement.

On a tout d'abord étudié la forme géométrique lu fond 3 de l'ampoule, telle qu'elle se présente sur plusieurs échantillons d'ampoules de 2 et 5 millilitres de contenance. Il a été constaté que le fond d'ampoule présente un rayon de courbure variant selon la distance du point de mesure de ce rayon à axe 4 de l'ampoule, entre quelques millimètres et l'infini. Ce fond d'ampoule se raccorde avec le cylindre d'ampoule suivant un faible rayon convexe 3a. Ce rayon 3a ne permettant pas de transmettre correctement de la lumière au liquide 2 depuis l'extérieur de l'ampoule 1, il ne sera pas mis à profit pour éclairer le liquide.

Des relevés montrent que le diamètre $\underline{d}$ de fond d'ampoule résiduel comportant la forme concave, ou "diamètre utile", correspond approximativement à une proportion fixe du diamètre extérieur de l'ampoule, quelle que soit l'ampoule.

On a déterminé par ailleurs, par mesures et calculs, le rayon de courbure moyen du fond d'ampoule, de façon à assimiler celui-ci à un système optique simple, à savoir un dioptre air-liquide divergent à courbure unique. On a constaté que, d'une ampoule à l'autre, ce rayon de courbure moyen varie. La description ci-dessous montre comment le dispositif de l'invention permet de s'affranchir, pour une large part, de ces variations du rayon de courbure.

Après avoir étudié le système optique constitué par les fonds d'ampoule, il va être maintenant expliqué comment l'on peut en tirer parti. Le faisceau lumineux idéal pour éclairer le liquide 2 aurait, pendant son trajet entre le fond 3 de l'ampoule 1 et le ménisque 6 formé par la surface libre du liquide, une enveloppe cylindrique épousant par faitement la paroi latérale intérieure 9 de l'ampoule 1, de façon à éclairer la totalité du liquide 2. Cependant, et comme expliqué précédemment, le fond de l'ampoule présente un rayon circulaire périphérique 3a qui n'est pas exploitable. En conséquence, un faisceau lumineux théorique va être défini, qui tient compte de cette limitation.

Sur la figure 1 a été tracée l'enveloppe 5 du faisceau lumineux théorique permettant, selon l'invention, d'éclairer le liquide 2 de façon optimale. Ce faisceau provient d'une source de lumière non représentée et située à gauche de l'ampoule 1, de façon que le faisceau pénètre dans l'ampoule 1 à partir de son fond 3 et selon la direction de son axe longitudinal 4. Ce faisceau converge vers le fond 3 de façon à traverser celui-ci suivant le diamètre utile $\underline{d}$ relatif à la zone concave du fond. Au-delà, le faisceau diverge légèrement pour rejoindre en ligne droite le cercle formé par le ménisque 6 de la surface libre du liquide 2 s'appuyant contre la paroi intérieure 9 de l'ampoule 1. Cette surface libre constitue la surface terminale du liquide précitée, par laquelle sort le faisceau. De cette façon, la quasi-totalité du volume du liquide est traversée par le faisceau lumineux, à l'exception de la zone circulaire hachurée 7, et ce, sans que le faisceau ne rencontre la portion de paroi latérale de l'ampoule en contact avec le liquide. Au-delà du ménisque 6, le faisceau peut indifféremment diverger et rencontrer la zone supérieure de paroi d'ampoule, comme représenté en figure 1. En effet, l'observation du liquide éclairé à partir du fond 3 s'effectue habituellement selon un axe 8 perpendiculaire à l'axe 4 d'éclairement, et il est connu de masquer tout rayonnement provenant de la zone d'ampoule située au-dessus du liquide, ou provenant de la source de lumière elle-même.

Avantageusement, le dispositif d'éclairement est conçu de façon que l'image de la source de lumière à travers ce dispositif et le fond d'ampoule 3 soit situé dans le plan du ménisque 6. En effet, l'image de la source correspond à une zone de convergence du faisceau, au-delà de laquelle celui-ci diverge. Aussi, si cette image se forme avant le ménisque 6, le faisceau rencontrera les zones de paroi de l' ampoule en contact avec le liquide, et sera à l'origine d'erreurs de détection. Si au contraire, cette image se forme au-delà du ménisque 6, le rendement lumineux au sein du liquide en sera diminué. Toutefois, cette deuxième configuration est envisageable.

La figure 2 représente le faisceau lumineux 10 issu du dispositif optique selon l'invention qui, lorsqu'il sera placé en regard du fond de l'ampoule, s'épanouira selon la forme définie en figure 1. Le fond de l'ampoule étant un dioptre divergent, le faisceau 10 sera convergent vers le fond d'ampoule de façon que, en association avec lui, il produise un faisceau 5 (figure 1) dont l'enveloppe est quasi-parallèle à la paroi latérale du récipient. On a figuré en 11 une source de lumière non ponctuelle et circulaire. Son faisceau de sortie 12 diverge selon un certain angle. Un dispositif de mise en forme du faisceau 12, ou système optique, dont on n'a représenté que les faces théoriques d'entrée et de sortie 13 et 13', capte le faisceau 12, de façon à récupérer si possible toute la lumière issue de la source 11. Le système optique est tel qu'au-delà de la face théorique de sortie 13', la lumière sorte selon le faisceau 10 qui converge vers une image 14 de la source 11, et qui s'épanouit en divergeant, au-delà de cette image. Le faisceau 10 doit, en coopérant avec le fond de l'ampoule, procurer le faisceau 5 (figure 1) et produire une nouvelle image au niveau du ménisque 6. Un tel faisceau a été défini par trois paramètres: la distance $D_1$ entre la face théorique de sortie 13' du second système optique et le plan 15 sur lequel reposera ultérieurement le fond de l'ampoule, la distance $D_2$ entre le plan 15 et l'image 14, et enfin le diamètre $\varphi$ de l'image 14. La connaissance de ces paramètres permettra de déduire la constitution réelle du dispositif d'éclairement.

La distance $D_1$ peut être choisie identique pour tous les types d'ampoules devant être contrôlées, de façon que le diamètre du faisceau 10 à la traversée du plan 15 corresponde au diamètre utile d'ampoule $\underline{d}$ le plus grand. Ainsi, pour de telles ampoules, il ne sera pas nécessaire d'utiliser un diaphragme pour réduire le diamètre du faisceau 10 au diamètre utile $\underline{d}$.

La distance $D_2$ a été choisie en ne tenant pas compte du rayon de courbure du fond d'ampoule, donc en considérant que ce fond est plat, et de façon que l'image 14 se situe, au travers d'une telle ampoule, dans le plan du ménisque du liquide. Dans la pratique, la distance $D_2$ est fonction de la hauteur de remplissage h et de l'indice de réfraction du liquide.

C'est enfin par la détermination du diamètre $\varphi$ de l'image 14 que l'on a pris en compte le rayon de courbure des ampoules réelles. Il a été choisi le plus grand possible, en fonction des possibilités du dispositif d'éclairement, de façon à éclairer au mieux le liquide, et ce pour une ampoule de rayon de courbure le plus petit, tel que celui-ci ressort des mesures et calculs mentionnés ci-dessus à partir d'un lot d'ampoules représentatif. Ainsi, le faisceau lumineux à l'intérieur de l'ampoule est celui le plus divergent qui puisse être produit. La détermination pratique de la valeur optimale de φ est réalisée graphiquement pour une taille donnée d'ampoules. Elle se déduit par homothétie pour d'autres tailles d'ampoules. Il faut noter, qu'ainsi déterminée, la valeur de φ permet l'éclairement de toutes les ampoules d'une même taille car celles-ci ont, par définition, un fond de rayon de courbure moyen plus grand que le rayon pris comme référence. Le faisceau qui traverse le fond diverge donc dans une moindre mesure et est donc toujours contenu à l'intérieur du récipient, même si l'éclairement est un peu moins large. Le dispositif d'éclairement selon l'invention ne nécessite donc pas de réglage systématique pour s'adapter aux caractéristiques pourtant variables des ampoules.

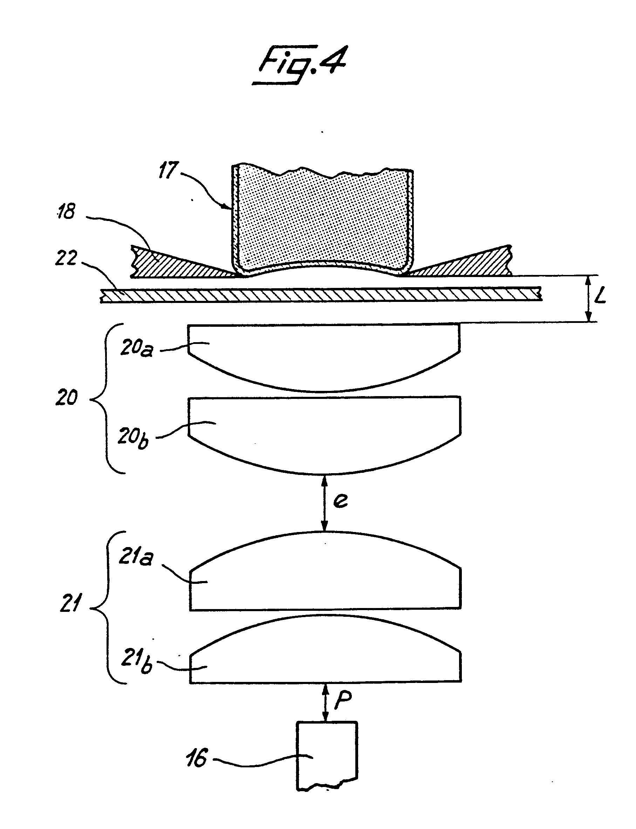

La figure 3 représente l'ampoule 1 placée devant la source 11 et le système optique figuré par les faces théoriques d'entrée et de sortie 13 et 13'. La source 11, le système optique 13 et 13' et l'ampoule 1 sont disposés coaxialement. Il en résulte (fig. 2), sans l'ampoule, un faisceau 10 de caractéristiques $(D_1, D_2, \varphi)$ définies précédemment. Toutefois, il est nécessaire d'adapter le diamètre du faisceau 10 au diamètre utile $\underline{d}$ de l'ampoule, de façon à éviter tout éclairement inutile et néfaste, et à obtenir la forme de faisceau conique représentée en figure 1. Aussi place-t-on un diaphragme circulaire 19 dans le plan 15 du fond d'ampoule, dont le diamètre d'ouverture est égal à $\underline{d}$ (fig. 3).

C'est donc une portion de faisceau 10' qui traverse le liquide 2 et vient rejoindre la paroi latérale intérieure 9, à une distance $D'_2$ du plan 15 qui est égale à la hauteur de remplissage h. Ce faisceau 10' forme en cet endroit une image 14' de la source 11 qui possède un diamètre φ égal au diamètre intérieur de l'ampoule. Au-delà, le faisceau 10' diverge indifféremment.

Le diaphragme 19, s'il limite le faisceau entrant 10, ne modifie ni la distance $D_2$, ni le diamètre φ définis précédemment et en conséquence ne perturbe pas la marche du faisceau 10' qui en résulte.

Un dispositif d'éclairement apte à produire un faisceau lumineux comme défini en figure 2 peut comporter une fibre optique, dont une extrémité est reliée à une source lumineuse primaire ou source chaude, et dont l'autre extrémité constitue la source froide du dispositif d'éclairement.

De préférence, la source 11 est une source secondaire formée à partir d'une source primaire non représentée, au travers d'un premier système optique condenseur également non représenté. Le système optique 13, 13' représenté est alors un second système optique formant l'image 14' de la source secondaire 11. Le premier système optique peut notamment comporter une fibre optique, dont l'entrée est reliée à la source primaire ou source chaude, et dont la sortie constitue la source secondaire 11, ou source froide.

Cet agencement permet avantageusement d'éloigner la source chaude dissipant des calories, des ampoules à contrôler dont le liquide ne risque donc pas de s'échauffer. De plus, plusieurs fibres optiques peuvent partir de la source chaude pour éclairer différentes ampoules disposées sur un support commun et contrôlées tour à tour ou simultanément. L'entrée de la fibre optique est reliée à la source chaude par un système optique qui condense, de façon connue en soi, la lumière issue de la source vers cette entrée; ce système optique comprend notamment un miroir et une ou plusieurs lentilles. A la sortie de la fibre optique, est placée au moins une lentille permettant de capter l'essentiel du faisceau sortant de la fibre optique, et de faire converger ce faisceau vers le fond de l'ampoule. Un diaphragme est disposé entre cette lentille et le fond de l'ampoule.

Différents moyens de réglage de ce dispositif d'éclairement sont possibles. On peut rendre réglables la distance entre la sortie de la fibre optique et la lentille disposée devant cette sortie et/ou la distance entre cette lentille et le fond de l'ampoule. Par ailleurs, le diamètre du diaphragme peut être aussi réglable.

Avantageusement, on peut remplacer la lentille unique disposée devant la sortie de la fibre optique par au moins deux lentilles ou groupes de lentilles non juxtaposées. Cet agencement permet d'introduire un paramètre de réglage supplémentaire de ce système optique, qui est la distance entre ces lentilles ou groupes de lentilles.

Sur la figure 4 est représenté un dispositif d'éclairement adapté aux dimensions d'ampoules pharmaceutiques de 2 à 5 millilitres à fond concave. Ses caractéristiques sont liées à un mécanisme de contrôle d'ampoules existant sur lequel il a dû s'adapter. Dans le cadre d'un nouveau projet global, ce dispositif pourrait être conçu différemment. Il comporte une sortie de fibre optique 16 disposée selon l'axe de l'ampoule 17 positionnée à distance au-dessus de la sortie 16, l'entrée de la fibre optique étant reliée à une source lumineuse primaire non représentée, telle qu'une lampe à filament. L'ampoule 17 repose sur un support 18 évidé circulairement selon le diamètre utile de l'ampoule et jouant avantageusement le rôle de diaphragme. Entre la sortie de fibre 16 et le support 18 sont disposées successivement, suivant l'axe de l'ampoule, deux lentilles prévues, pour des raisons de commodité, sous la forme de deux doublets (20, 21) de lentilles élémentaires planconvexe (20$\underline{a}$, 20$\underline{b}$; 21$\underline{a}$, 21$\underline{b}$). Les deux lentilles de chaque doublet sont juxtaposées; leurs faces convexes sont orientées dans le même sens et en regard de celles de l'autre doublet.

Une vitre de protection 22 est disposée entre la lentille 20$\underline{a}$ voisine du fond d'ampoule et celui-ci, de façon à protéger le système optique qui est par ailleurs enfermé dans un boîtier non représenté, tout en laissant passer le faisceau lumineux. L'écartement e entre les deux doublets de lentilles, ainsi que l'écartement p entre la lentille 21b et la sortie de la fibre 16 sont réglables, par des moyens appropriés. Leur valeur, déterminée par un calcul numérique tenant compte des conditions énoncées précédemment, peut être affinée après expérimentation.

Le dispositif d'éclairement selon l'invention augmente efficacement le niveau d'éclairement maximal à l'intérieur de l'ampoule, niveau au-delà duquel les défauts ou les inscriptions de la paroi provoquent des détections erronées: par exemple la sortie de la fibre optique 16 placée seule devant le fond de l'ampoule ne permet pas de dépasser un niveau d'éclairement de 90 000 lux au sein du liquide, sur l'axe et à 1 cm du fond de l'ampoule. En utilisant le dispositif selon l'invention, cet éclairement passe à 450 000 lux, apportant un gain égal à 5. Cela signifie que la surface observée de chaque particule solide au sein du liquide est 5 fois mieux éclairée. La quantité de lumière reçue par le dispositif détecteur de particules étant proportionnelle à la surface de ces particules, on peut aussi dire qu'on pourra détecter une particule de surface 5 fois plus faible, c'est-à-dire de diamètre plus de deux fois plus petit qu'avec la fibre optique seule.

Le système optique décrit dans l'exemple ci-dessus, n'est pas unique. Il dépendra, dans une large mesure, du type de récipients à éclairer. De façon générale, le dispositif d'éclairement selon l'invention peut être constitué par tout système optique permettant de créer, à partir de la source, l'image 14 de la figure 2. Ce système peut être réalisé à l'aide de miroirs, de lentilles ou de toute combinaison de ces deux composants. Le choix des composants intervenant dépend du principe adopté mais aussi d'impératifs théoriques ou techniques non directement liés, comme par exemple la nécessité de modifier la forme de la source, le choix d'une gamme différente de longueurs d'ondes ou l'utilisation de miroirs si les flacons sont de diamètre important.

Une amélioration notable peut être obtenue en adaptant la forme de la source à la forme du récipient, comme évoqué précédemment.

La souplesse du système optique peut être avantageusement améliorée dans certains cas, en plaçant un diaphragme limitant l'étendue du faisceau, à la traversée du fond du récipient, comme dans l'exemple décrit.

Le dispositif à fibre optique décrit précédemment concerne d'abord l'éclairement de petites ampoules. Comme expliqué précédemment, son adaptation à de grands flacons peut être réalisée, jusqu'à un certain point, en augmentant notamment le diamètre de la fibre. Au-delà, il faut concevoir un autre dispositif, illustré par la figure 5.

Sur cette figure est représenté un flacon cylindrique 30 de grandes dimensions (sa contenance est par exemple égale à un litre). Le flacon 30 dessiné horizontalement est en réalité disposé verticalement, son fond 31 se trouvant en partie supérieure. Il est rempli partiellement d'un liquide 32 à contrôler, ce liquide reposant sur le goulot 33 et le bouchon du flacon 30 et présentant une surface libre 34 faisant face au fond 31.

Différents éléments optiques sont disposés du côté du fond du flacon 30, coaxialement au flacon 30 dont l'axe est désigné par la référence 46. Un premier système optique permet de concentrer la lumière d'une source primaire 35, constituée par exemple par un filament de lampe à iode, sur un premier diaphragme 36 définissant la source secondaire.

Ce système, placé entre la source 35 et le premier diaphragme 36, comprend un groupe de deux lentilles, soit une lentille asphérique 37 correspondant sensiblement à une demi-sphère, et une lentille plan-convexe 38. Le diamètre de ces lentilles est sensiblement supérieur à celui du premier diaphragme 36. Ces lentilles sont disposées en regard, à savoir que leurs convexités sont tournées l'une vers l'autre. Le premier système optique comprend encore, à proximité de la source 35, un miroir concave 39 en forme de calotte sphérique dont le diamètre a des dimensions analogues à celles des lentilles précitées. Ce miroir 39 est disposé coaxialement au groupe de lentilles 37, 38, du côté de la source 35 opposé à la lentille asphérique 37, sa concavité étant en regard de cette lentille. Le centre de courbure du miroir 39 coïncide avec la source 35.

Les moyens de mise en forme du faisceau lumineux comprennent, outre le groupe de lentilles 37, 38, le miroir 39 et le premier diaphragme 36, une lentille complémentaire 40 constituant le second système optique. La lentille complémentaire 40 est plan-convexe et son diamètre est égal au diamètre utile du flacon 30. Sa convexité est tournée vers le fond 31 du flacon 30 et disposée à une distance appropriée de ce fond. Un second diaphragme 41 peut éventuellement limiter la largeur du faisceau au niveau du fond du récipient. Ce diaphragme peut être confondu avec le support de la lentille complémentaire 40 ou un des moyens de positionnement du flacon 30. L'éloignement entre la lentille complémentaire 40 et la lentille plan-convexe 38 du groupe de lentilles est de l'ordre de grandeur de la somme des distances focales de ces deux lentilles.

En fonctionnement, la lentille complémentaire 40 coopérant avec le dioptre plan constitué par la surface libre 34 du liquide 32 contenu dans le flacon 30 donne, du premier diaphragme 36 constituant la source secondaire, une image 42 située, soit au niveau de la face terminale du liquide, soit, comme représenté sur la figure 5 au-delà. La surface terminale du liquide, à partir de laquelle le faisceau lumineux sort du liquide, est constituée ici par la surface par laquelle le liquide 32 s'appuie sur le goulot 33 du flacon 30; la surface libre 34 du liquide 32 est la surface d'entrée du faisceau dans ce liquide. Dans cet exemple, l'image 42, a un diamètre égal au diamètre intérieur du flacon 30. Le faisceau, entre les limites constituées d'une part par le second diaphragme 41 et d'autre part par l'image définitive 42 du premier diaphragme 36, présente une enveloppe cylindrique, le second diaphragme 41 et l'image définitive ayant un diamètre égal au diamètre intérieur du flacon 30. La figure 5 montre la marche d'un rayon 43 coïncidant avec l'enveloppe cylindrique.

Ce rayon 43 issu de la source 35 délimite le cône d'émission de lumière de la source 35 capté par la lentille asphérique 37, ce cône d'émission étant augmenté du cône qui lui est opposé et qui est réfléchi par le miroir 39. Le rayon 43 fortement divergent est redressé par le groupe de lentilles 37, 38; il se dirige ensuite vers le bord de l'ouverture du premier diaphragme 36, puis vers un point de convergence 44 représentant le point image théorique du filament source 35, l'ouverture du premier diaphragme 36 définissant une image intermédiaire de la source primaire 35; le rayon 43 rencontre ensuite la zone périphérique de la lentille complémentaire 40; il traverse celle-ci, passe près du bord de l'ouverture du second diaphragme 41 et poursuit alors un chemin parallèle à l'axe du flacon 30, traverse le fond 31 et longe la face intérieure de la paroi du flacon 30, en étant adjacent à cette face. Le trajet optique du rayon 43, comme d'ailleurs celui de tous les autres rayons traversant le fond 31, n'est pas modifié sensiblement par ce fond, lequel n'est pas en contact avec le liquide 32 et se comporte comme une lame à faces parallèles.

Le rayon 43 rencontre ensuite la surface libre 34 du liquide, ou surface d'entrée dans le liquide. Celle-ci étant très proche d'un plan, elle ne modifie pas le trajet du rayon 43; en revanche, dans le cas d'un flacon de petit diamètre, la surface libre en forme de ménisque risquerait de perturber la marche des rayons dans le liquide 32 car elle constituerait un dioptre air-liquide divergent. Dans ce dernier cas, la modification de la convergence et/ou de la position de la lentille complémentaire 40 permettrait de tenir compte de la présence de ce dioptre, analogue à celui constitué par le fond des ampoules pharmaceutiques.

Enfin, le rayon 43 poursuit son trajet en ligne droite, au sein du liquide 32, puis hors du flacon 30.

Un second rayon lumineux 45 permet de préciser la position axiale et le diamètre de l'image 42 du premier diaphragme 36. Ce rayon 45 vient frapper la lentille complémentaire 40 en un point opposé au point correspondant du rayon 43. Il traverse obliquement le flacon 30, et définit, au-delà de ce flacon, l'enveloppe du faisceau lumineux qui, alors diverge. Le point d'intersection entre les deux rayons 43 et 45, au-delà du flacon 30, définit la position axiale et le diamètre de l'image 42.

Naturellement, l'invention s'applique aussi à l'éclairement de récipients de grandes dimensions disposés fond en bas. Dans ce cas, il faudra tenir compte de l'influence du fond du récipient sur le faisceau lumineux, comme expliqué en détail en regard des figures 1 à 3. Grâce à un choix approprié de la distance focale et de la position du second système optique, on produira une convergence du faisceau entre le second système optique et le fond du récipient, de façon à obtenir, au sein du liquide, un faisceau d'enveloppe quasi-cylindrique.

L'invention trouve notamment son application dans la détection de défauts dans les liquides alimentaires et pharmaceutiques.

**Revendications**

1. Dispositif d'éclairement d'un liquide transparent contenu dans un récipient (1) transparent au rayonnement utilisé et dont les parois latérales sont parallèles à l'axe longitudinal (4; 46) dudit récipient (1), en vue du contrôle du liquide, comportant des moyens (19) pour positionner le récipient (1), une source lumineuse pour éclairer le liquide à partir du fond (3) du récipient (1) et selon ledit axe (4; 46), et des moyens (13, 19), pour mettre en forme le faisceau lumineux (12) issu de la source lumineuse, disposés sur le trajet du faisceau entre celle-ci et le fond du récipient (1) et coopérant avec le système optique constitué par la surface du liquide par laquelle le faisceau entre dans celui-ci de façon à produire au sein du récipient (1) un faisceau d'enveloppe quasi-cylindrique et de façon à former une image (14') de la source lumineuse à travers les moyens (13, 19) de mise en forme et ladite surface d'entrée dans le liquide, caractérisé en ce que les moyens (13, 19) de mise en forme sont agencés pour produire seuls un faisceau (10) dont la vergence a une valeur qui est fonction de celle de la surface d'entrée dans le liquide assimilée à une surface à rayon de courbure unique, ladite vergence étant de signe opposé à celui de la vergence de ladite surface d'entrée, la section (d) du faisceau, observée dans un plan (15) situé au droit du fond du récipient, ayant sensiblement les dimensions et la forme de la section intérieure du récipient (1), ce faisceau formant une image (14) de la source lumineuse d'une section ($\varphi$) telle et située à une distance ($D_2$) telle dudit plan (15) en aval de celui-ci qe, lorsque le récipient (1) est disposé par son fond dans ledit plan (15), le faisceau traverse sensiblement tout le volume du liquide sans rencontrer les zones de paroi latérale du récipient (1) en contact avec le liquide, et l'image (14') de la source lumineuse à travers les moyens (13, 19) de mise en forme et la surface d'entrée dans le liquide soit située au voisinage de la surface terminale du liquide.

2. Dispositif selon la revendication 1, caractérisé en se que, dans le cas de contrôles successifs dans un grand nombre de récipients d'un type donné, disposés de façon que le liquide prenne appui sur le fond des récipients, la section (φ) de ladite image (14) formée par les seuls moyens (13, 19) de mise en forme, est choisie de façon que ceux-ci produisent, en coopération avec la surface d'entrée dans le liquide d'un récipient prelevé parmi un lot de récipients représentatif, comme présentant un fond dont le rayon de courbure moyen est le plus petit, un faisceau dont la section, au voisinage de la surface terminale du liquide, a les dimensions de celles de la section intérieure du récipient.

3. Dispositif selon la revendication 1 ou la revendication 2, caractérisé en ce que, dans le cas du contrôle dans un récipient dont le fond (3) présente une partie concave se raccordant auxdites parois latérales par une partie (3a) convexe et qui est disposé de façon que le liquide prenne appui sur le fond du récipient, la section (d) du faisceau produit par les seuls moyens de mise en forme (13, 19), observée dans ledit plan (15), a les dimensions de ladite partie concave du fond.

4. Dispositif selon la revendication 1, caractérisé en ce que ladite source lumineuse (36) est une source secondaire, dont les dimensions sont compatibles avec celles du récipient (30) et qui est formée à partir d'une source primaire (35), au travers d'un premier système optique (37, 38, 39), lesdits moyens de mise en forme du faisceau issu de la source lumineuse (36) étant constitués par un second système optique (40) formant, en coopération avec ladite surface d'entrée dans le liquide (34), ladite image (42) de la source secondaire (36) située au voisinage de la surface terminale du liquide (32).

5. Dispositif selon l'une quelconque des revendications précédentes, caractérisé en ce que le ou les systèmes optiques comprennent chacun au moins une lentille (37, 38, 40) et/ou un miroir (39).

6. Dispositif selon la revendication 4, caractérisé en ce que, dans le cas de l'éclairage d'une ampoule pharmaceutique (17) de faible contenance, ledit premier système optique comporte une fibre optique (16) de section homothétique de celle de ladite ampoule (17) et disposée selon l'axe de celle-ci, dont l'entrée reçoit le faisceau issu de ladite source primaire et dont la sortie constitue ladite source secondaire, et en ce qu'il comporte un diaphragme (18) de section sensiblement égale à la section intérieure de ladite ampoule (17), qui est disposé entre ledit second système optique (20, 21) et le fond de l'ampoule (17).

7. Dispositif selon la revendication 6, caractérisé en ce que ladite ampoule (17) est disposée verticalement, fond (3) en bas, la surface terminale susdite étant alors constituée par la surface libre (6) du liquide (2).

8. Dispositif selon la revendication 4, caractérise en ce que, dans le cas de l'éclairage d'un flacon (30) de grande contenance, ledit premier système optique (37, 38, 39) concentre le faisceau sur un premier diaphragme (36) de section homothétique de celle dudit flacon (30), qui est disposé entre les deux systèmes optiques et qui constitue ladite source secondaire, et en ce qu'il comprend un second diaphragme (41) de section sensiblement égale à la section intérieure du flacon (30), qui est disposé entre ledit second système optique (40) et le fond du flacon (30).

9. Dispositif selon la revendication 8, caractérisé en ce que ledit flacon (30) est disposé verticalement, fond en haut, la surface terminale susdite étant alors constituée par la surface d'appui du liquide sur une autre paroi d'extrémité (33) du flacon.

10. Dispositif selon la revendication 8, caractérisé en ce que lesdits moyens de mise en forme du faisceau comprennent un miroir plan de renvoi qui est disposé en travers dudit faisceau, entre ledit premier groupe optique (37, 38, 39) et le fond du flacon (30).

**Patentansprüche**

1. Vorrichtung zur Beleuchtung einer transparenten Flüssigkeit, die in einem bezüglich der verwendeten Strahlen transparenten Behälter (1) enthalten ist, dessen Seitenwände in Blickrichtung der Prüfung der Flüssigkeit gegenüber seiner Längsachse (4, 46) parallel sind, mit Mitteln (19) zum Positionieren des Behälters (1), mit einer Lichtquelle zur Beleuchtung der Flüssigkeit vom Boden (3) des Behälters (1) aus und entlang besagter Längsachse (4, 46), und mit Mitteln (13, 19) zum Aufbereiten des von der Lichtquelle ausgehenden Lichtbündels (12), die im Verlauf des Bündels zwischen dieser Lichtquelle und dem Boden des Behälters (1) angeordnet sind und die mit dem von der Flüssigkeitsoberfläche, durch die das Lichtbündel eintritt, gebildeten optischen System zusammenwirken, um innerhalb des Behälters (1) ein quasi-zylindrisches Hüllbündel zu erzeugen und um ein Bild (14') der Lichtquelle durch die Mittel (13, 19) zum Aufbereiten und durch die besagte Eintrittsfläche der Flüssigkeit hindurch zu erzeugen, dadurch gekennzeichnet, daß die Mitteln (13, 19) zum Aufbereiten ausgelegt sind, um alleine ein Bündel (10) zu erzeugen, dessen Kon- oder Di-vergenz einen Wert hat, der abhängig von demjenigen der Eintrittsfläche in die Flüssigkeit ist, die einer Fläche mit definiertem Krümmungsradius entspricht, wobei die besagte Kon- oder Di-vergenz in umgekehrtem Sinn gegenüber der Di- oder Kon-vergenz der Eintrittsoberfläche ist, dass der Querschnitt (d) des Bündels, gesehen in einer lotrecht zum Boden des Behälters liegenden Ebene (15), im wesentlichen die Abmessungen und die Form des Innendurchmessers des Behälters (1) hat, dass dieses Bündel ein Bild (14) der Lichtquelle mit einem entsprechenden Querschnitt (φ) bildet, das sich in einer entsprechenden Entfernung ($D_2$) stromauf von

besagter Ebene (15) befindet, dass, wenn der Behälter (1) mit seinem Boden in besagter Ebene (15) angeordnet ist, das Bündel im wesentlichen das ganze Volumen der Flüssigkeit durchdringt, ohne auf die Zonen der mit der Flüssigkeit in Kontakt befindlichen Seitenwände des Behälters (1) aufzutreffen, und daß das Bild (14') der Lichtquelle durch die Mittel (13, 19) zum Aufbereiten und durch die Eintrittsfläche hindurch in die Flüssigkeit im Bereich der Endfläche der Flüssigkeit gelegt wird.

2. Vorrichtung nach Anspruch 1, dadurch gekennzeichnet, daß im Falle von aufeinanderfolgenden Untersuchungen einer großen Zahl von Behältern eines vorgegebenen Typs, die derart angeordnet sind, daß sich die Flüssigkeit auf dem Boden des Behälters abstützt, der Querschnitt (φ) des besagten Bildes (14), das nur durch die Mittel (13, 19) zum Aufbereiten gebildet wird, derart gewählt wird, daß diese zusammen mit der Eintrittsfläche in die Flüssigkeit eines Behälters, der eine Menge von repräsentativen Behältern entnommen wird, weil er einen Boden mit geringstem mittlerem Krümmungsradius aufweist, ein Bündel liefern, dessen Querschnitt im Bereich der Endfläche der Flüssigkeit die Dimension von denen des Innenquerschnitts des Behälters hat.

3. Vorrichtung nach Anspruch 1 oder 2, dadurch gekennzeichnet, daß im Falle der Untersuchung in einem Behälter, dessen Boden (3) einen konkaven Bereich aufweist, der mit den Seitenwänden über einen konvexen Bereich (3a) verbunden ist und der derart angeordnet ist, daß sich die Flüssigkeit auf den Boden des Behälters abstützt, der Querschnitt (d) des Bündels, der nur durch die Mittel (13, 19) zum Aufbereiten erzeugt wird, und der in besagter Ebene (15) betrachtet wird, die Dimensionen von besagtem konkaven Bereich des Bodens hat.

4. Vorrichtung nach Anspruch 1, dadurch gekennzeichnet, daß besagte Lichtquelle (36) eine Sekundärquelle ist, deren Abmessungen denen des Behälters (30) angepaßt sind und die ausgehend von einer Primärquelle (35) über ein erstes optisches System (37, 38, 39) gebildet wird, wobei die besagten Mittel zum Aufbereiten eines von der Lichtquelle (36) ausgehenden Bündels mit einem zweiten optischen System (40) gebildet sind, das zusammen mit der besagten Eintrittsfläche (34) der Flüssigkeit das besagte Bild (42) der Sekundärquelle (36) bildet, das im Bereich der Endfläche (32) liegt.

5. Vorrichtung nach einem der vorangehenden Ansprüche, dadurch gekennzeichnet, daß das oder die optischen Systeme jeweils wenigstens eine Linse (37, 38, 40) und/oder einen Spiegel (39) enthalten.

6. Vorrichtung nach Anspruch 4, dadurch gekennzeichnet, daß im Falle der Beleuchtung einer pharmazeutischen Ampulle (17) mit geringem Inhalt das besagte erste optische System eine Faseroptik (16) mit homothetischem Querschnitt zu der Ampulle (17) aufweist, die in deren Achse angeordnet ist und deren Eintritt das von der Primärquelle ausgehende Bündel empfängt und deren Austritt die Sekundärquelle bildet, und daß diese eine Blende (18) mit einem im wesentlichen dem Innenquerschnitt der Ampulle (17) gleichen Querschnitt enthält, die zwischen dem besagten zweiten optischen System (20, 21) und dem Boden der Ampulle (17) angeordnet ist.

7. Vorrichtung nach Anspruch 6, dadurch gekennzeichnet, daß die besagte Ampulle (17) vertikal mit dem Boden (3) nach unten angeordnet ist, wobei die Endfläche dann von der freien Oberfläche (6) der Flüssigkeit (2) gebildet wird.

8. Vorrichtung nach Anspruch 4, dadurch gekennzeichnet, daß im Falle der Beleuchtung eines Flakon (30) mit großem Inhalt das besagte erste optische System (37, 38, 39) das Bündel mit einer ersten Blende (36) mit homothetischem Querschnitt zu besagtem Flakon (30) einengt, die zwischen den beiden optischen System angeordnet ist und die die besagte Sekundärquelle bildet, und daß sie eine zweite Blende (41) mit einem im wesentlichen dem Innenquerschnitt des Flakon (30) gleichen Querschnitt enthält, die zwischen dem besagten zweiten optischen System (40) und dem Boden des Flakon (30) angeordnet ist.

9. Vorrichtung nach Anspruch 8, dadurch gekennzeichnet, daß der Flakon (30) senkrecht mit dem Boden nach oben angeordnet ist, wobei die erwähnte Endfläche dann durch die Abstützfläche der Flüssigkeit auf einer anderen Endwand (33) des Flakon gebildet wird.

10. Vorrichtung nach Anspruch 8, dadurch gekennzeichnet, daß die besagten Mittel zum Aufbereiten des Bündels einen planen Reflexionsspiegel enthalten, der quer zum Bündel zwischen der ersten optischen Gruppe (37, 38, 39) und dem Boden des Flakon (30) angeordnet ist.

**Claims**

1. Apparatus for illuminating a transparent liquid contained in a receptacle (1) which is transparent to the radiation used, for purposes of controlling the quality of the liquid, the receptacle (1) having its side walls parallel to the longitudinal axis (4; 46) of the said receptacle (1), wherein the apparatus includes means (19) for positioning the receptacle (1), a luminous source for illuminating the liquid from the base (3) of the receptacle (1) in a direction corresponding with the said axis (4; 46), and means (13, 19) for shaping the beam of light (12) propagated from the light source and disposed in the path of the beam between the latter and the base of the receptacle (1) whereby to cooperate with the optical system constituted by the surface of the liquid through which the beam enters into the liquid, and so as to produce in the interior of the receptacle (1) a beam having a quasi cylindrical envelope, whereby also to form an image (14') of the light source through the beam shaping means (13; 19) and the said surface of entry into the liquid, the apparatus being characterised in that the beam shaping means (13, 19) are adapted to produce by themselves a beam (10) the convergence or divergence of which has a value which is a function of that of the surface of its entry into the liquid equivalent to a surface having a single radius of curvature, the said convergence or divergence being of opposite sign to the divergence or convergence of the said surface of entry, with the cross section (d) of the beam, when observed in a plane (15) situated square to the base of the receptacle, having substantially the dimensions and the shape of the internal cross section of the receptacle (1), so that the said beam forms an image (14) of the light source in a cross section ($\varphi$) situated at a distance ($D_2$) downstream of the said plane (15), the said cross section ($\varphi$) and the said distance ($D_2$) being such that, when the receptacle (1) is disposed with its base in the said plane (15), the beam passes through substantially the whole volume of the liquid without meeting the parts of the side wall of the receptacle (1) which are in contact with the liquid, and being also such that the image (14') of the light source through the beam shaping means (13, 19) and the surface of its entry into the liquid are situated in the vicinity of the terminal surface of the liquid.

2. Apparatus according to claim 1, characterised in that for successive quality control operations in a large number of receptacles of a given type, so disposed that the liquid is in contact with the base of the receptacles, the cross section ($\varphi$) for the said image (14), formed by the beam shaping means (13, 19) by themselves, is so chosen that the latter will produce, in cooperation with the surface of entry of the beam into the liquid in a receptacle selected as having the base with the smallest mean radius of curvature out of a group of representative receptacles, a beam such that in the vicinity of the terminal surface of the liquid, the dimensions of the cross section of this beam are those of the internal cross section of the receptacle.

3. Apparatus according to Claim 1 or Claim 2, characterised in that, for quality control in a receptacle the base (3) of which has a concave portion joined to the said side walls through a convex portion (3a), with the receptacle disposed in such a way that the liquid is in full contact with the base of the receptacle, the cross section (d) of the beam produced by the beam shaping means (13, 19) by themselves, when observed in the said plane (15), has the dimensions of the said concave portion of the base.

4. Apparatus according to Claim 1, characterised in that the said light source (36) is a secondary source, the dimensions of which are compatible with those of the receptacle (30), with the secondary light source receiving light from a primary light source (35) through a first optical system (37, 38, 39), the said means for shaping the beam propagated from the light source (36) comprising a second optical system (40) which forms, in cooperation with the said surface (34) of entry of the beam into the liquid, the said image (42) of the secondary light source (36) situated in the vicinity of the terminal surface of the liquid (32).

5. Apparatus according to any one of the preceding Claims, characterised in that the or each of the said optical systems comprises at least one lens (37, 38, 40) and/or mirror (39).

6. Apparatus according to Claim 4, characterised in that, for illuminating a pharmaceutical ampoule (17) of small capacity, the said first optical system includes an optical fibre (16) of the said ampoule (17) disposed coaxially with respect to the latter, the input end of the optical fibre being such as to receive the beam propagated from the said primary source, with the output end of the optical fibre constituting the said secondary source, and in that the apparatus further includes a diaphragm (18) having a cross section substantially equal to the internal cross section of the said ampoule (17), the diaphragm being disposed between the said second optical system (20, 21) and the base of the ampoule (17).

7. Apparatus according to Claim 6, characterised in that the said ampoule (17) is disposed vertically with its base (3) downwards, the said terminal surface then being constituted by the free surface (6) of the liquid (2).

8. Apparatus according to Claim 4, characterised in that, for the illumination of a flask (30) of large capacity, the said first optical system (37, 38, 39) is arranged to concentrate the beam on to a first diaphragm (36) having a cross section corresponding to that of the said flask (30), the first diaphragm (36) being disposed between the two optical systems and constituting the said secondary source, and in that the apparatus further includes a second diaphragm (41) having a cross section which is substantially equal to the internal cross section of the flask (30), the second diaphragm (41) being disposed between the said second optical system (40) and the base of the flask (30).

9. Apparatus according to Claim 8, characterised in that the said flask (30) is disposed vertically, upside down, the said terminal surface then comprising the surface of contact of the liquid on an end wall (33) of the flask other than the base thereof.

10. Apparatus according to Claim 8, characterised in that the said means for shaping the beam include a flat reflecting mirror which is disposed across the said beam, between the first said optical group (37, 38, 39) and the base of the flask (30).

*Fig.1*

*Fig.2*

*Fig.3*

_Fig.4_

_Fig.5_

EP 0 144 275 B1